# EUROPEAN PATENT APPLICATION

(11) **EP 1 616 536 A2**
(43) Date of publication of application: **18.01.2006**
(21) Application number: 05110421.4
(22) Date of filing: 06.05.1998
(51) Int. Cl.: A61F 2/06

(54) **TMR stent and delivery system**

(30) Priority: 08.05.1997 US 45991 P; 08.05.1997 US 45992 P; 08.05.1997 US 46003 P; 08.05.1997 US 46866 P; 29.01.1998 US 73309 P
(62) Divisional of application: 98201480.5
(71) Applicant: C.R. BARD, INC., Murray Hill New Jersey 07974 (US)
(72) Inventor: Gambale, Richard E., Tyngsboro, MA 01879 (US)
(74) Representative: van Westenbrugge, Andries

(57) **Abstract**

A device and method for revascularizing myocardial tissue of the heart using stents are provided. Multiple stents are implanted into an area of hibernating myocardial tissue to permit blood to flow from the left ventricle directly into the tissue for revascularization. Various stent embodiments are disclosed that simultaneously pierce and penetrate the tissue during delivery and that are configured to resist migration out of the tissue once implanted. Alternatively, the stents may be inserted into myocardial tissue over a piercing delivery device. The stents are preferably delivered through a steerable catheter percutaneously insertable into the left ventricle and configured to hold multiple stents. The stents are pushed from the catheter directly into the myocardium sequentially.

## Description

### Field of the Invention

This invention relates to a system for revascularizing myocardial tissue. Such a system comprises stents placeable in tissue to provide patent channels for revascularization of the tissue. The invention also relates to a system for delivering the stents to the intended site within a patient.

### Background of the Invention

The concept of revascularizing the myocardium by creating channels into the heart muscle to allow blood to flow directly from the left ventricle has gained increasing acceptance in recent years. It has been known that creating channels partially into the myocardium permits blood from the ventricle to reach sinusoids within the muscle. The restoration of blood flow, revascularization, to dormant or hibernating tissue can restore the muscle's normal pumping function, if the tissue has remained viable despite the previous deprivation of blood. Revascularizing the myocardium by creating passages into the tissue through which blood may flow has become known as Transmyocardial Revascularization (TMR).

Early researchers, more than thirty years ago, reported promising results for revascularizing the myocardium by piercing the muscle to create multiple channels for blood flow. (Sen, P.K. et al., "Transmyocardial Acupuncture - A New Approach to Myocardial Revascularization", *Journal of Thoracic and Cardiovascular Surgery,* Vol. 50, No. 2, August 1965, pp. 181-189). Although others have reported varying degrees of success with various methods of piercing the myocardium to restore blood flow to the muscle, many have faced common problems such as closure of the created channels. Various techniques of perforating the muscle tissue to avoid closure have been reported by researchers. These techniques include piercing with a solid sharp tip wire, hypodermic tube and physically stretching the channel after its formation. Reportedly, many of these methods still produced trauma and tearing of the tissue that ultimately led to closure of the channel.

An alternative method of creating channels that potentially avoids the problem of closure involves the use of laser technology. Researchers have reported success in maintaining patent channels in the myocardium by forming the channels with the heat energy of a laser. (Mirhoseini, M. et al., "Revascularization of the Heart by Laser", *Journal of Microsurgery,* Vol. 2, No. 4, June 1981, pp. 253-260). The laser was said to form channels in the tissue were clean and made without tearing and trauma, suggesting that scarring does not occur and the channels are less likely to experience the closure that results from healing.

All of the above approaches to TMR as surgical access to the heart. Although there has been some published recognition of the desirability of performing TMR in a catheterization procedure, there does not appear to be evidence that such procedures have been put into practice. For example, U.S. Patent No. 5,429,144 (Wilk) discloses inserting an expandable stent within a preformed channel created within the myocardium for the purposes of creating blood flow into the tissue from the left ventricle. Aita patents 5,380,316 and 5,389,096 disclose another approach to a catheter based system for TMR.

A document which falls under the provisions of Art. 54(3) EPC, WO 97/44071, discloses an intraventricular pump which is disposed at a distal end of a catheter, which is introduced into the left ventricle of the heart. The intraventricular pump comprises tubes that swing radially outwards and protrude from a hub after the pump has been pushed out of the catheter. The tips of the tubes pierce the endocardium and myocardium and become embedded in the myocardium. Thus, the tubes become anchored to the myocardium such that they can act to inject blood into the myocardium wall from the ventricle.

### Summary of the Invention

The TMR stent and associated delivery system of the present invention provide a simplified mechanism for creating revascularizing channels in the myocardium by implanting self-piercing stents directly into ischemic tissue. The invention comprises a self-piercing stent delivered into the myocardium to stimulate blood flow directly to the tissue from the left ventricle.

Because the stents are self-piercing, a separate channel need not be formed into the tissue prior to stent placement. The stents are rigid and expansion from a reduced delivery diameter to a larger diameter for implantation into a preformed channel is not necessary. Rather, a sharp distal tip enables each stent to pierce the tissue and imbed itself when it is pushed into the myocardium. The stents may have a length that corresponds to a penetration depth approximately equal to three-quarters of the thickness of the myocardium. A flange may be included at the proximal end of the stent that catches the surface of the myocardium to ensure that the stent is not inserted too deeply into the tissue.

Outward migration of the stents after insertion is prevented by tissue engaging barbs formed on their outside surface. The barbs project outward at an acute angle to the side surface of the stent and point proximally. Therefore the barbs will not catch tissue during distal movement of the stent into the myocardium, but will claw into tissue if there is proximal movement of the stent out of the tissue.

The hollow interior of the imbedded stents provides a passageway through which blood may pass from the ventricle into the tissue. Blood entering the stent from its proximal end, open to the ventricle, perfuses into surrounding tissue through holes formed through the sidewall and/or distal end of the stent. Perfusion capability may be enhanced by piercing radially extending channels through enlarged side ports of the implanted stent using a sharp tip wire inserted through the proximal opening of the stent.

Alternatively, the stent can be formed from a flexible helically coiled spring. Various materials and cross-sectional shapes can be used for the spring ribbon that is wrapped into a helical shape. The overall shape of the coiled stent can be varied, though at least a portion of the stent should be tapered to the distal end to form a pointed tip that facilitates penetration into the myocardium.

Outward migration of the coiled stent after insertion is prevented by configuring the stent to have tissue engaging protrusions on its outside surface. As the ribbon used to form the coiled stent is wrapped about a mandrel, it may be canted away from parallel to the longitudinal axis of the stent to create a raised edge on each coil. The coils of the stent form a saw tooth outer surface on the stent. The mandrel upon which the ribbon is wrapped can be configured with a ribbed spiral surface that holds each coil in the canted configuration. Alternatively, the ribbon may be placed in tension during wrapping on a mandrel to cause the edges of the coils to curve outward and protrude from the stent body. Alternatively, a ribbon of trapezoidal cross-section can be wrapped about a straight mandrel so that the surface of each coil of the resulting spring has a raised edge. In these embodiments, the surface of each coil is configured to project outward at an angle to the longitudinal axis of the stent. Each coil has an outwardly projecting raised edge angled in the proximal direction. Therefore, the raised edges of the stent coils will not catch onto the tissue during distal movement of the stent into the myocardium, but will claw into tissue to resist proximal movement of the stent out of the myocardium occurs. Some embodiments of the wrapped coil stent may be sufficiently flexible to require support of a piercing delivery device positioned within the stent interior during implantation into the myocardium.

Preferably, the TMR stents are delivered to the heart transluminally by a delivery catheter, avoiding the need for surgery. The pointed stents may be configured to be nested within each other for tandem containment within the delivery catheter, thereby permitting delivery of stents at multiple locations in the myocardium with one catheterization. The delivery catheter may be guided to the left ventricle by tracking a previously inserted barbed tip guidewire anchored in the myocardium. After reaching the ventricle, the distal tip of the catheter may be steered to different points on the surface of the myocardium to deliver the several stents over an area of ischemic tissue. The pushing force needed to eject the stents from the distal end of the delivery catheter and into the myocardium may be exerted through a push wire that is slidable through the catheter. A restraint mechanism for preventing inadvertent discharge of the stents from the catheter may include a sheath having inwardly projecting resilient fingers to restrain the stent under light to moderate forces. Application of delivery force pushes the leading of the nested stents past the resilient fingers and into the tissue.

It is an object of the present invention to provide a stent insertable into tissue such as the myocardium of the heart that is configured to maintain a blood passageway directly into the tissue for revascularization.

It is another object of the invention to provide a stent insertable into tissue that is self-piercing and resists migration after insertion.

It is another object of this invention to provide a stent delivery system capable of carrying a single stent or multiple stents to the myocardium of a patient percutaneously and transluminally.

It is yet another object of this invention to provide a stent delivery system capable of providing a pushing force sufficient to insert the stent into the myocardium.

### Brief Description of the Drawings

The foregoing and other objects and advantages of the invention will be appreciated more fully from the following further description thereof, with reference to the accompanying diagrammatic drawings wherein:
FIG. 1 is a diagrammatic, sectional illustration of the left ventricle of the human heart having several stents of the present invention implanted within the myocardial tissue;
FIG. 2A is a side view of a molded TMR stent having a taper only at the distal end;
FIG. 2B is a side view of a molded TMR stent having a full taper along its length;
FIG. 2C is a side view of a molded TMR stent having a full taper and body curvature along its length;
FIG. 2D is a diagrammatic longitudinal cross-section of a portion of the side wall of a molded stent;
FIG. 3 is a side view of a molded TMR stent having a full taper along its length and an enlarged side port;
FIG. 3A is a longitudinal cross-section of the molded TMR stent having interior passages for side ports that is implanted in myocardial tissue;
FIG. 3B is a longitudinal cross-section of the molded TMR stent with a piercing wire inserted through the interior channels and into the myocardial tissue;
FIG. 3C is a longitudinal cross-section of the molded TMR stent after additional flow channels have been created by the piercing wire;
FIG. 4A is a top view of a perforated flat sheet configured to be rolled to form a TMR stent;
FIG. 4B is a side view of a TMR stent formed by rolling a perforated flat sheet;
FIG. 4C is a sectional view of the perforated flat sheet taken along the line 4C-4C in FIG. 4A;
FIG. 4D is a side view of an alternate embodiment of the rolled TMR stent having an enlarged side port;
FIG. 5A is side view of the self-piercing TMR stent formed from a wound metal ribbon;
FIG. 5B is a longitudinal cross-section of the self-piercing TMR stent formed from a wound metal ribbon;
FIG. 6A is side view of the canted coil spring TMR stent;
FIG. 6B is a longitudinal cross-section of the canted coil spring TMR stent;
FIG. 6C is a side view of a section of the TMR stent wrapped on a mandrel;
FIG. 7A is a cross-sectional side view of a TMR stent having a distal taper;
FIG. 7B is a cross-sectional side view of a TMR stent having a full taper;
FIG. 7C is a cross-sectional side view of a TMR stent having an increased diameter midsection and a distal taper
FIG. 7D is a cross-sectional view of a TMR stent having a curved full taper.
FIG. 8A is a side view of a coil spring TMR stent formed from a ribbon having a trapezoidal cross-section;
FIG. 8B is a longitudinal cross-section of a coil spring TMR stent formed from a ribbon having a trapezoidal cross-section;
FIG. 8C is a detailed cross-sectional view of several coils of a TMR stent formed from a ribbon having a trapezoidal cross-section;
FIG. 9A is a cross-sectional side view of several canted coils of a TMR stent;
FIG. 9B is a side view of a coil spring TMR stent;
FIG. 9C is a side view of a coil spring TMR stent placed on a piercing delivery device;
FIG. 9D is a front view of a coiled TMR stent placed on a piercing delivery device;
FIG. 10 is a diagram of the barbed tip guidewire being delivered to the left ventricle of the heart through a guide catheter;
FIG. 11A is a longitudinal cross-sectional view of the delivery catheter for TMR stents;
FIG. 11 B is an axial cross-sectional view of the delivery catheter taken along the line A-A in FIG. 11A;
FIG. 12A is a longitudinal cross-section of the distal tip of the delivery catheter carrying several TMR stents;
FIG. 12B is a longitudinal cross-sectional view of the delivery catheter delivering TMR stents into the myocardium;
FIG. 12C is a longitudinal cross-sectional view of the delivery catheter after having delivered a TMR stent into the myocardium;
FIG. 13 is a longitudinal cross-sectional view of the delivery catheter delivering a TMR stent with flow channels into the myocardium;
FIGS. 14A-14D illustrate the steps of delivering several TMR stents to an area of myocardial tissue in the left ventricle.

### Description of the Illustrative Embodiments

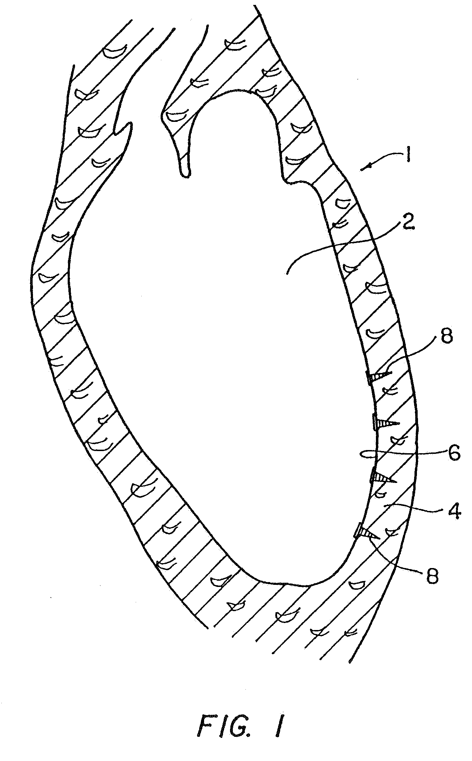

FIG. 1 shows, in diagrammatic section, the left ventricle 2 of a human heart 1. The TMR stents 8 of the present invention are implanted through the endocardial surface 6 into myocardial tissue 4 to provide pathways for blood to flow directly from the ventricle into the tissue for the purpose of revascularization. Blood flowing from the ventricle through the hollow stent 8 perfuses into the tissue surrounding the stent. The perfusion of blood revitalizes ischemic tissue, if that tissue has remained viable despite the previous deprivation of blood.

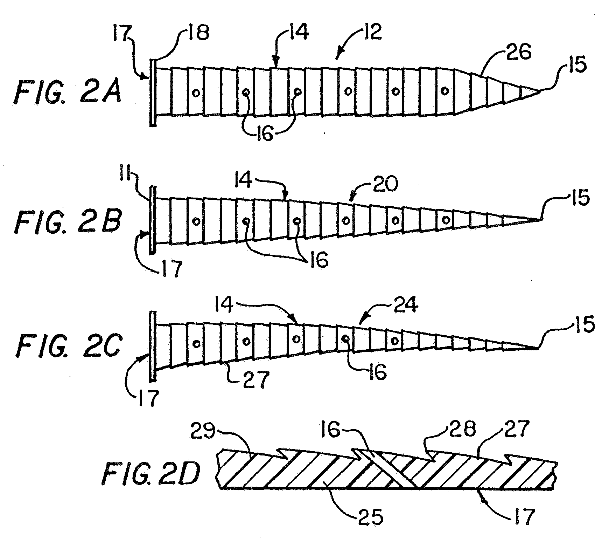

In a preferred embodiment, the TMR stent 8 is cylindrically or conically shaped having a hollow interior 17 for blood flow and a pointed distal end for piercing and embedding into myocardial tissue. FIG. 2A shows a preferred TMR stent 12 that is molded having a body 14 that is straight with a distal taper 26 and perfusion holes 16. Alternatively, a molded stent 20 may have a body 14 that is straight with full taper as shown in FIG. 2B. A third variation of a molded stent 24 may have a body 14 that is conical and has a slight curvature along its longitudinal axis with a full taper, similar to a quill, as shown in FIG. 2C. A flange 18 located at the proximal end of the stents becomes flush with the endocardial surface of the myocardium upon implantation to control insertion depth of the stent. Though shown in the figures as closed, the pointed distal tip 15 may be open to provide another perfusion hole through the stent.

The conical shape of the stents shown in FIGS. 2A-2C is significant in that it permits multiple stents to be nested, one distal end arranged within the proximal end of the next. The pointed end of each stent may reside partially within the open cone shape interior 17 of the next stent to permit multiple stent delivery through a sheath or catheter. Additionally, the pointed configuration of the stent enables penetration of the myocardial tissue, without prior formation of a channel into the tissue.

The molded TMR stent is preferably formed from a plastic material such as high density polyethylene (HDPE). The length of the stent may be approximately 1.0 centimeter and is intended to correspond to a penetration depth of approximately 75% of the thickness of the myocardium. The maximum outside diameter of the body of the tapered stent is on the order of .060"-.070" (0.1524 - 0.1778 centimeter). The diameter of the flange portion 18, on the order of .080" -.100" (0.2032 - 0.254 centimeter), is larger than the body portion to catch the endocardial surface of the myocardium and prevent the stent from being inserted too deeply. The force required to insert the stents into the myocardium is on the order of 0.2 pounds (0.89 Newton).

Circumferential ridges 27 formed along the length of the molded stents serve as small proximally facing barbs 28. In FIG. 2D, a detailed cross section of the side wall of the molded of FIGS. 2A-2C is shown. The barbs project outward in a proximal direction, at an angle acute to the side surface 29 of the stent so as not to hamper insertion. However, the orientation of the barbs causes them to grip the surrounding tissue to prevent the stent from migrating in a proximal direction out of the myocardium after implantation. Perfusion holes 16 may be formed throughout the body 14 of the stent to permit blood flow, entering the interior 17 of an implanted stent from the ventricle, to flow radially outward into the myocardial tissue. The perfusion holes are on the order of .004" - .010." (0.01016 - 0.0254 centimeter) in diameter and may pass through the stent side wall at an angle, as shown in FIG. 2D, or may be perpendicular to the longitudinal axis of the stent. The number of holes may be varied but it is believed that blood flow is improved by maximizing their quantity. Other shapes of perfusion outlets, such as slots or spaces, could also be used.

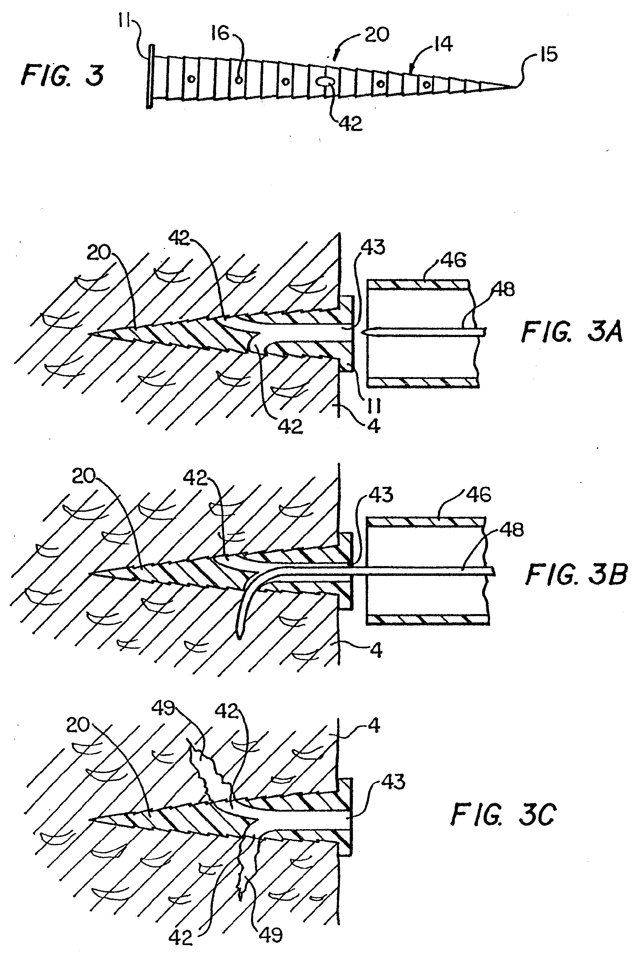

FIG. 3 shows a preferred molded TMR stent 20 having a body 14 that is straight with a full taper, and having perfusion holes 16 and enlarged side port 42 to increase blood flow out of the stent. Blood flow from the stents into the myocardium may be further enhanced by puncturing channels into the tissue surrounding the implanted stent. FIGS. 3A through 3C, are longitudinal cross-sectional views of implanted molded stents 20 with directing channels 43 joining the enlarged side ports 42 to the open proximal end 11 of the stent. A piercing tube or wire 48 is guided distally through the delivery catheter 46, into the directing channels 43 which direct the piercing tip to the enlarged side ports 42 to penetrate the myocardium 4 (FIG 3B). The piercing wire should be introduced through the stent immediately after implantation while still in alignment with the delivery catheter.

As represented in FIG. 3C, the piercing tube creates radial channels 49 in the myocardium extending from the side ports 42. Creation of the radial channels 49 forms a defined, continuous blood pathway to the myocardium 4 from the ventricle 2 via the directing channels 43 and side ports 42 of the stent. It is believed that the defined pathway extended by the radial channels 49 promotes increased blood flow into the myocardium.

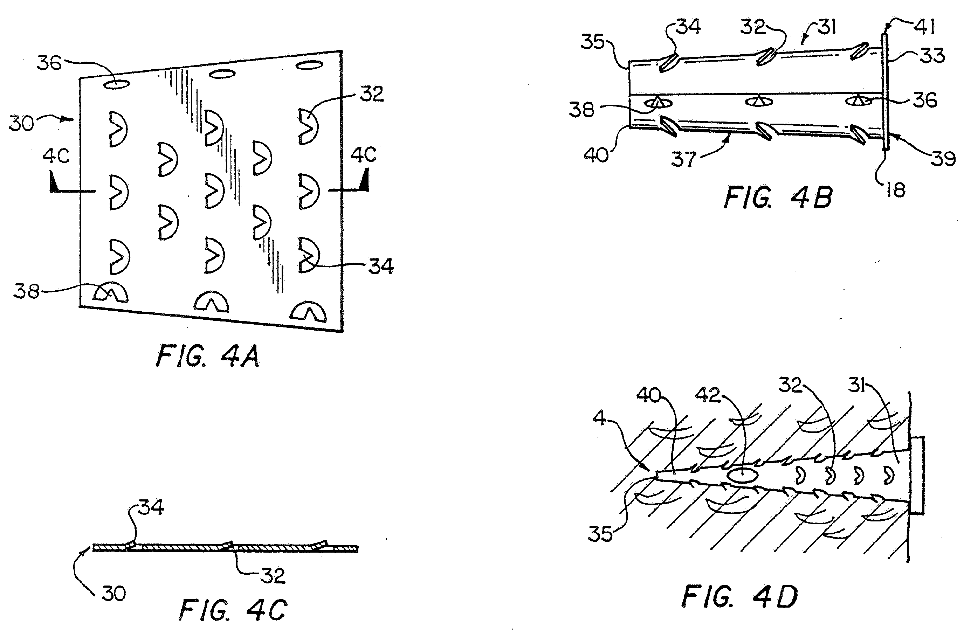

Another embodiment of the TMR stent 31 has a cylindrically or conically shaped body 37 having a hollow interior 39 for blood flow and a pointed distal end 40 for piercing and embedding into myocardial tissue. The TMR stent is fabricated by rolling a perforated metal sheet 30, shown in FIG. 4A, into a tapered, tubular shape stent 31 shown in FIG. 4B. The metal sheet 30 may be a material such as 304 stainless steel having a thickness on the order of .0005" - .003" (0.00127 - 0.00762 centimeter). The stent also may be formed from a sheet of suitable plastic material such as HDPE. Perforations 32 may be etched or stamped into the sheet while in a flat configuration. Perforations 32 are shaped to have a triangular barb 34. After formation of the perforations 32, the triangular barb shapes 34 may be bent out of plane from the metal sheet as shown by the sectional view shown in FIG. 4C. The bent triangular shapes 34 serve to locate the implanted stent as projecting barbs. The perforations 32 serve as perfusion outlets through which blood can flow out of the stent. FIG. 4D shows an implanted rolled TMR stent 31 having an enlarged side port 42 for increased blood flow into the myocardial tissue 4.

Eyelets 36 and locking tabs 38 are formed at the edges of the flat sheet to provide a mechanism for holding the rolled sheet in a tubular configuration. As shown in FIG. 4B, once the sheet is rolled, locking tabs 38 hook into eyelets 36, locking in place under the resilient expansion force of the rolled sheet. The length of the stent is approximately 1.0 centimeter, maximum outside diameter is on the order of .060"-.070" (0.1524 - 0.1778 centimeter) and the diameter of the flange portion 18 is, on the order of .080" - .100" (0.2032 - 0.254 centimeter). The tapered tubular shape of the stent provides a proximal opening 33, through which blood enters from the ventricle, and an opening 35 at the piercing distal end 40 to provide an additional perfusion outlet into the tissue. A flange 41 at the proximal end of the stent can help maintain stent depth in the myocardium such that the proximal opening 33 is in fluid communication with the left ventricle.

Raised barbs 34 project outward from the tubular stent at an acute angle to the axis of the stent, pointing towards the proximal end of the stent. The angular orientation of the barbs 34 permits insertion of the stent into tissue (in the distal direction) but prevents migration of the stent back out of the tissue in the proximal direction, as the barbs will hook into the tissue.

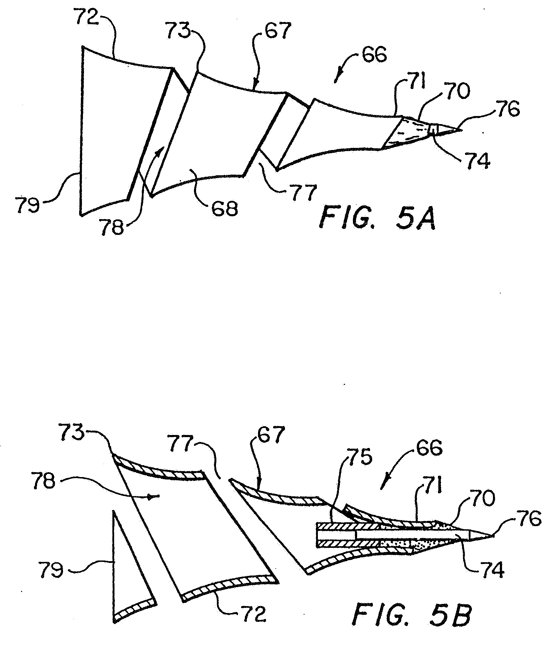

In a another embodiment, a TMR stent 66 comprises a body 67 formed from a helically wound flat ribbon 68 as shown in Fig. 5A. The ribbon is formed into a conical shape by wrapping it around a tapered mandrel (not shown) to cause plastic deformation. The ribbon 68 is wound with an increasing diameter as it extends proximally, providing the stent with a generally conical shape and hollow interior 78. A suitable material for the ribbon is 304 or 316 stainless steel having a thickness of approximately .003" (0.00762 centimeter) and a width of approximately .005" - .015" (0.0127 - 0.0381 centimeter)

As best seen in Fig. 5B, the distal end 71 of the ribbon is bonded at joint 70 by soldering, brazing or welding to a central mandrel 74 that extends partially through the interior of the stent. A stainless steel mandrel may be used having a diameter of approximately .012" (0.03048 centimeter). The distal tip 76 of the mandrel is sharpened to provide the stent with a piercing tip capable of penetrating the myocardium. The proximal end of the mandrel is bonded to a short segment of hypodermic tubing 75 to facilitate nesting of additional stents for multiple stent delivery. The hypotube may have an outside diameter of approximately .020" (0.0508 centimeter) and inside diameter of approximately .015" (0.0381 centimeter) to provide a suitable receptacle for the distal tip of another stent nested behind. The proximal end 79 of the ribbon may be soldered, brazed or welded upon itself to maintain the conical, wrapped configuration. Delivery force is applied in a distal direction, internally to the stent through the hypotube 75 and central mandrel 74. Spaces 77 between the wrappings of the ribbon permit blood to flow from the interior of the stent outward into the surrounding tissue of the myocardium.

To form barb-like projections on the outside surface of the stent, the stainless steel ribbon material may be placed in tension during the initial winding process to deform the ribbon to have a curved cross-sectional shape as is shown in FIG. 5B. The concave surface 72 of the ribbon creates flared edges 73 which act as barbs to grasp the tissue once the stent is implanted.

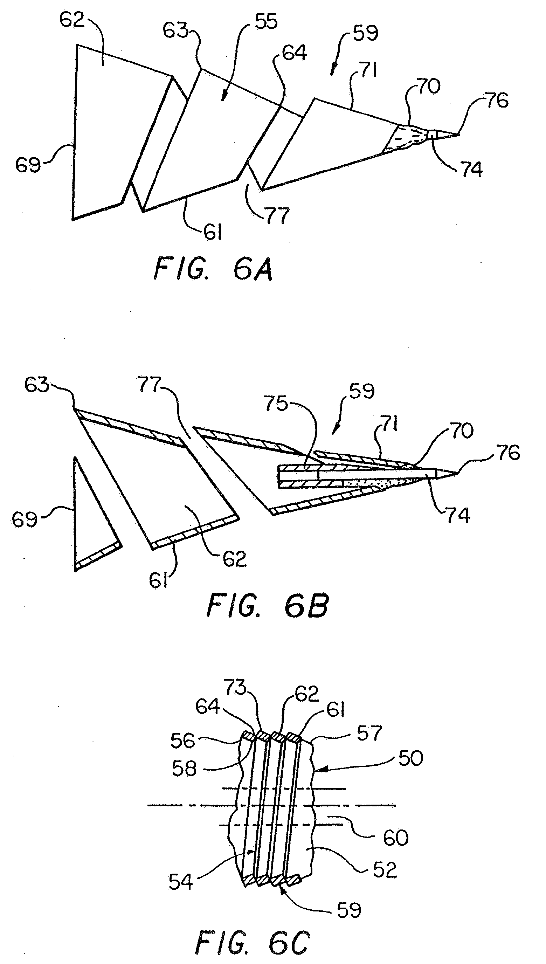

Alternatively, a helically wrapped stent 59 shown in Figs. 6A and 6B can be formed by wrapping the ribbon 61 around a mandrel 50 having a ribbed spiral surface 52 to cause plastic deformation of the ribbon as shown in Fig 6C. Wrapping the ribbon around the mandrel forms a stent body 55 comprised of several helically arranged coils 62 spaced slightly apart. The spaces 77 between the coils 62 of the ribbon permit blood to flow from the interior 78 of the stent outward into the surrounding tissue of the myocardium also serve to make the stent more flexible so that it can move with the myocardial tissue.

The wrapping mandrel 50 has several annular ribs 54 around its surface 52 that serve to hold each coil 62 of the ribbon 61. Each rib 54 has a frusto-conical surface 57 having a small diameter portion 58 and a large diameter portion 56. The frusto-conical surface 57 of each rib 54 extends away from the longitudinal axis of the mandrel at an acute angle. The ribbon 61, has a rectangular cross-section. The principal axis of the rectangular cross section is maintained at an acute angle to the longitudinal axis of the mandrel about which the ribbon is deformed. As the ribbon 61 is wrapped about the mandrel, each coil 62 fits into a rib 54 and is maintained at the acute angle that the frusto-conical surface makes with the longitudinal axis of the wrapping mandrel 50. The resulting coiled ribbon presents a raised edge 63 at the proximal side of each coil. As each coil presents a raised edge 63 and a recessed edge 64, a saw tooth surface configuration is formed along the length of the stent to resist forces that tend to push the stent back out of the myocardium after it has been inserted. The orientation of the canted coils presents the recessed edge 64 as the leading edge when the stent is moved in the distal direction such as during insertion into the tissue. However, the raised edge 63 is presented as the leading edge if the stent is urged proximally out of the tissue so that the edge becomes anchored onto the tissue to resist movement.

The wrapping mandrel 50 may be made dissolvable so that it can be easily removed from the interior 78 of the completed spring stent 59 about which it has been wrapped. Because each coil 62 of the stent is wrapped firmly into the ribs 54, sliding or unwinding the stent off the mandrel is difficult. A dissolvable mandrel can be broken down and washed away from within the completed stent by application of a solvent. The structure of the dissolvable mandrel can be fortified during stent wrapping by a removable rigid core (not shown). The core is inserted through a central bore 60 of the mandrel to provide added stiffness while the stent is wrapped. After the stent has been formed around the mandrel, the core can be slid out from the bore 60 and solvent applied to the mandrel. The dissolvable mandrel can be machined or molded from polystyrene, with acetone as a suitable solvent.

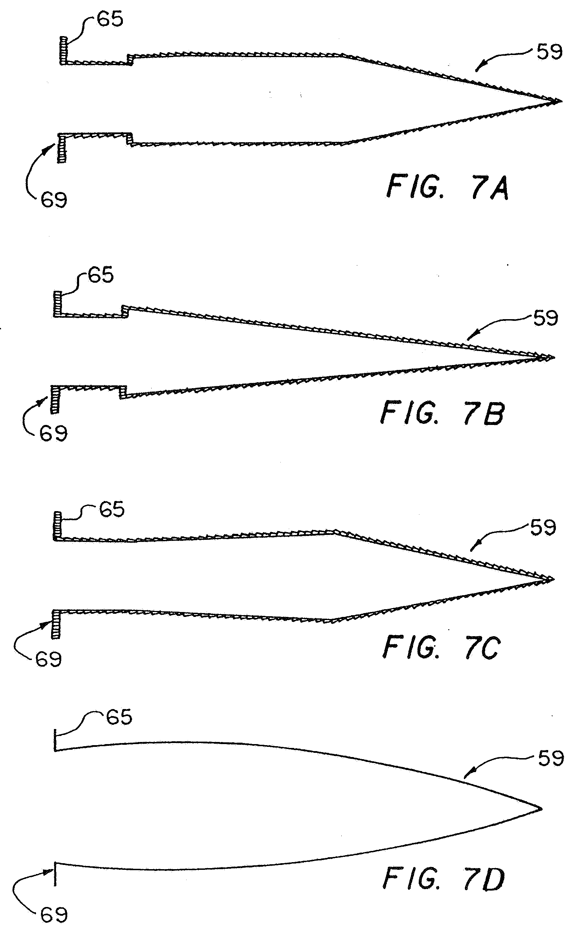

Preferably the ribbon 61 is wound with an increasing diameter as it extends proximally, providing the stent 59 with a generally conical shape. However, as shown in Figs. 7A - 7D various configurations of the conical shape are possible with the wrapped spring TMR stent embodiment. Various combinations of barrels, tapers, shoulders and arcs can be employed in the shape of the stent to make it easily insertable into the myocardium while also making it resistant to withdrawal. A preferred stent may be on the order of 0.35" (0.889 centimeter) in length and .06" (0.1524 centimeter) in maximum diameter. Additionally, as shown in Figs. 7A - 7D, the stent 59 may be formed with a flange 65 at its proximal end 69 to prevent insertion of the stent too deeply into the myocardium. The flange, having a wider diameter than the rest of the stent, is intended to become flush with the endocardial surface of the myocardium as the stent is inserted.

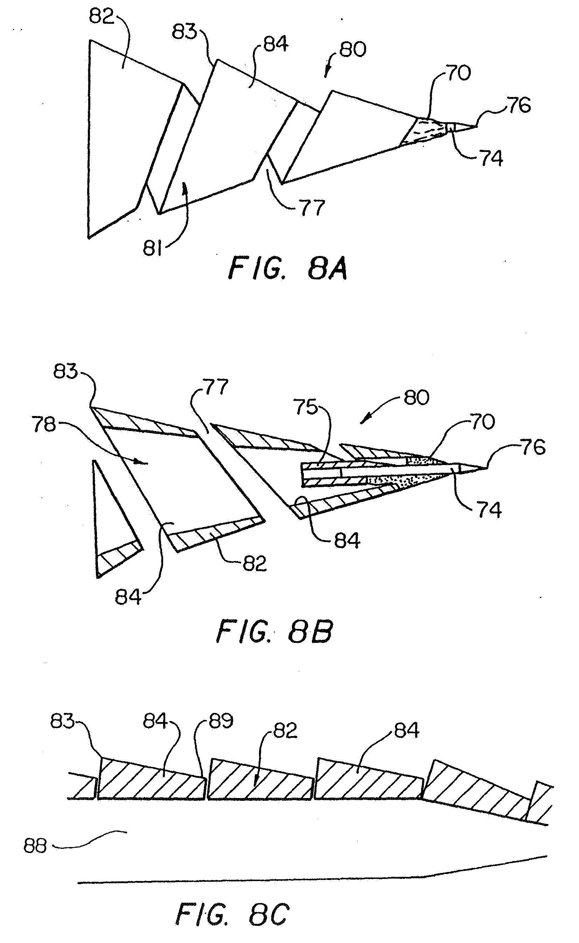

In an alternative wrapped spring stent embodiment 80 shown in Figs. 8A and 8B, a coil spring body 81 having a raised edge 83 at the proximal end of each coil 84 is formed by wrapping a ribbon 82 of trapezoidal cross-section around a smooth mandrel. Though the trapezoidal shape ribbon lies flat on the smooth mandrel 88, as shown in Fig 8C, the tapered thickness of the ribbon causes it to become canted so that the proximal edge 83 of each coil 84 to be raised further from the stent central axis than the distal coil edge 89. The raised edge 83 of each canted coil 84 serves to anchor the implanted stent in the myocardium similarly to the raised edges 63 of the coils of the previous stent embodiment. Trapezoidal 316 stainless steel winding wire is a suitable material for this stent embodiment. Dimensions of the trapezoidal cross-section should be on the order of 0.010" (0.0254 centimeter) wide with a maximum thickness on the order of 0.002" (0.00508 centimeter) and a minimum thickness on the order of 0.001" (0.00254 centimeter).

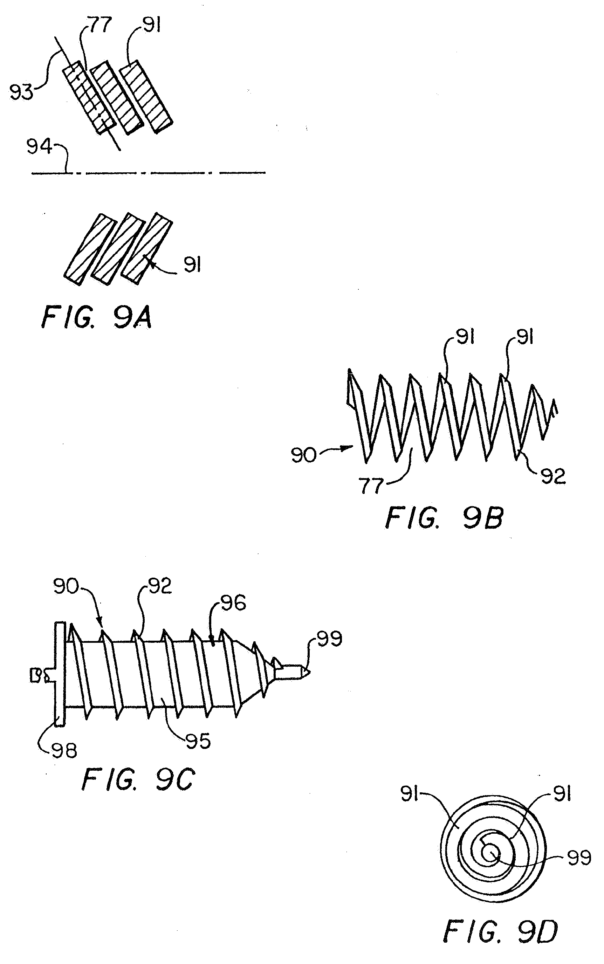

Figs. 9A - 9D show another wrapped spring stent embodiment 90. The stent 90 is formed by wrapping a rectangular cross-sectional wire around a ribbed mandrel, similar to the embodiment shown in FIG. 6C. However in the present embodiment, the long axis 93 of the rectangular cross-section ribbon is oriented substantially perpendicular to the longitudinal axis 94 of the stent, as is shown in Figs. 9A - 9D. In this configuration, the major axis 93 of the coils 91 of the rectangular ribbon 92 tend to extend radially from the longitudinal axis of the stent 90 at an acute angle. With greater coil surface area extending away from the longitudinal axis of the stent at an angle, the stent is believed to be more stable and less likely to migrate once implanted within the myocardium. The stent is preferably formed from 316 stainless steel rectangular cross-section forming wire. The stent 90 may be as long as 0.35" (0.889 centimeter) in length and as wide as 0.09" (0.2286 centimeter) in diameter.

Unlike the previous wrapped coil spring stent embodiments of Figs. 5A-8B, the stent of Figs. 9A-9D does not employ a central mandrel and hypotube. Therefore, the stent 90 is not readily nestable with other stents for multiple stent delivery, nor is it self-piercing. Instead, this stent embodiment is delivered one at a time while placed over a piercing mandrel delivery device 96 as shown in Figs. 9C and 9D. The delivery device comprises a body 95 to support the stent coils 92, a sharpened tip 99 for piercing the myocardium and a backstop 98 to keep the stent 90 from sliding off the back of the device 96 when the assembly is pushed into the myocardium. Though the present stent embodiment is not self-piercing, the delivery device simultaneously pierces the myocardium and implants a stent, as occurs during delivery of the other stent embodiments.

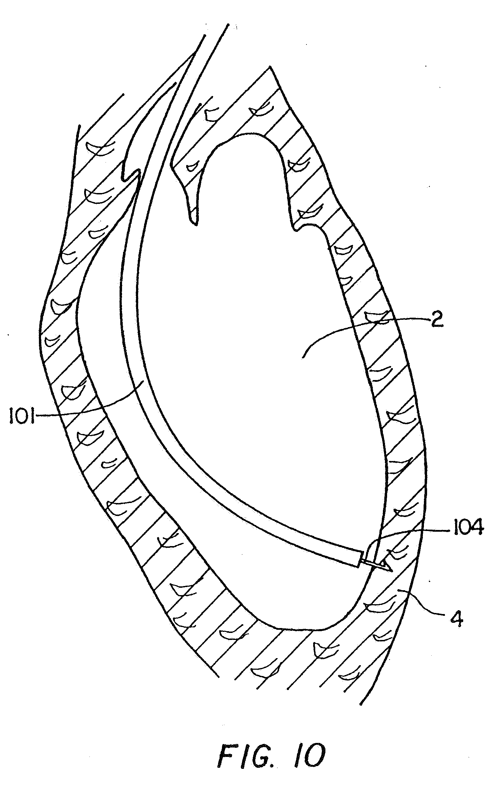

All TMR stent embodiments described above can be delivered to the myocardium percutaneously and transluminally through the left ventricle of the heart as shown diagrammatically in Fig. 10. The delivery system comprises a steerable catheter with an inner sheath and pusher wire to deliver multiple self-piercing stents. A barbed tip guidewire anchored in the myocardium guides the deflectable tip catheter to the intended area. The rotation of the catheter around the eccentrically oriented guidewire provides a circumferential area within which the delivery system can implant stents into the myocardial tissue. Up to ten stents or more may be implantable with one catheterization to revascularize an area of myocardial tissue. Because the stents delivered are self-piercing, no prior channel creation through the delivery catheter is required before stent delivery.

In use of the TMR stent delivery system a guide catheter 101 is first navigated through the patient's vessels to reach the left ventricle 2 of the heart as shown in FIG. 10. A barbed tip guidewire 104 is then inserted through the guide catheter and into the ventricle where it pierces the myocardium 4 and becomes anchored within the tissue. The guidewire 104 may be fabricated of stainless steel and have a diameter of approximately .020" (0.0508 centimeter). The barbed shape formed at the distal tip should be sharp enough to easily penetrate the myocardium but resist removal from the tissue at forces of at least up to 0.2 pounds (0.89 Newton) (the stent delivery force). A stop 115 is bonded near the distal end of the guidewire prevents excessive penetration into the myocardium.

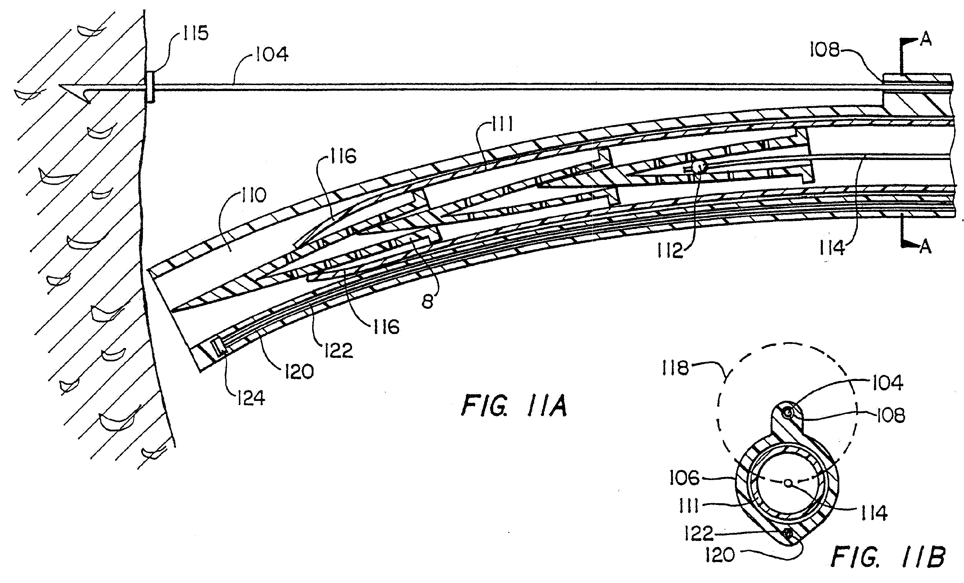

After the barbed tip guidewire is anchored in the myocardium, the guide catheter is withdrawn and a steerable stent delivery catheter 106 shown in FIG. 11A is advanced over the guidewire 104 to become positioned within the ventricle for stent delivery. The guidewire lumen 108 of the catheter is oriented eccentrically on the catheter 106. Therefore, when the catheter is rotated about the guidewire 104, the center of the catheter 106 rotates through a circular path 118 as is shown in FIG. 11 B to encompass a broader delivery area with one guidewire placement. The outside diameter of the delivery catheter is preferably less than .100" (0.254 centimeter).

Steering capability is provided by a pull wire 122 extending the length of the catheter in lumen 120 and terminating in a bond 124 near the distal tip of the catheter. Pulling the wire 122 from the proximal end causes the more flexible distal tip of the delivery catheter 106 to buckle, thereby providing steering control.

The deflectable tip catheter 106 has a large central lumen 110 which slidably receives a flexible inner sheath 111. The distal end of the flexible sheath has several resilient fingers 116 located around the circumference of the sheath projecting distally and inwardly toward the center of the lumen to restrain stents 8 loaded within the catheter. Within the inner sheath slides a push wire 114 having a ball 112 near its distal end for engaging the interior of a TMR stent 8

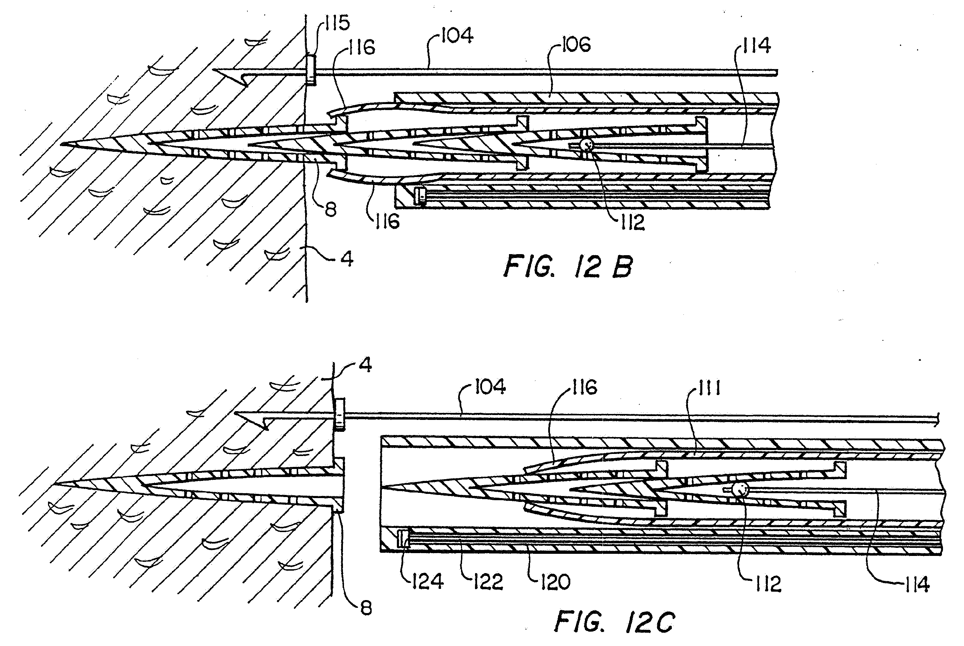

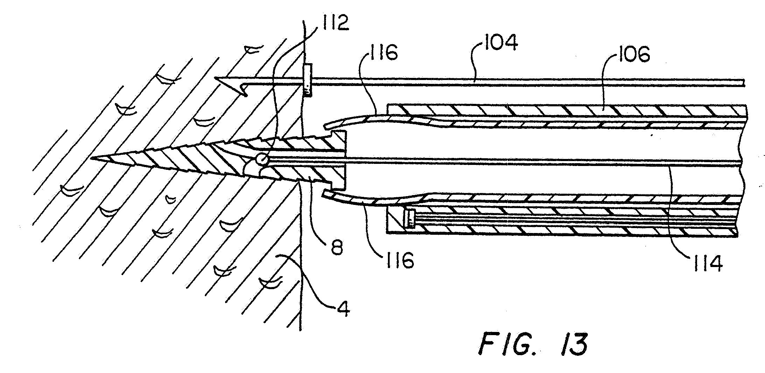

To deliver the stent 8 into the myocardium, the push rod 114 and inner sheath 111 are advanced distally in unison to move a stent 8 out of the catheter 106 as shown in FIG. 12B. The distal tip of the inner sheath 111 projects slightly from the catheter 106 during delivery. However, it is the push rod 114 and associated ball 112 pushing against the interior of the most proximal stent that continues distally, driving the leading (most distal) stent 8 into the myocardial tissue. However, the stents of the embodiment of FIGS. 3 - 3C cannot be nested due to directing channels formed in their interior and, thus are delivered singularly as shown in Fig. 13. The canted wrapped spring stent embodiment 90 shown in Figs. 9A - 9D, is also delivered singularly, through the delivery catheter, carried over the piercing delivery device 95 shown in Fig. 9C. Rather than being self-piercing like the other stent embodiments, the canted spring stent 90 is delivered over the delivery device 95 which simultaneously pierces the tissue and delivers the stent into the opened tissue. For all stent embodiments, a force of approximately 0.2 pounds (0.89 Newton) should be sufficient to embed the stent embodiments into the myocardium.

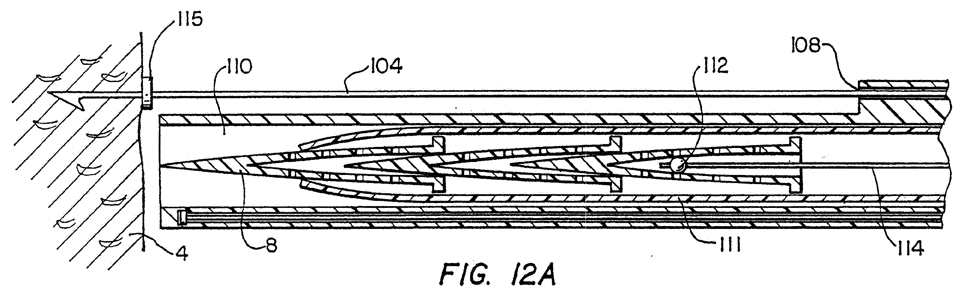

As is shown in FIGS. 12A - 12C, the barbed guidewire 104 locates the catheter 106 in position and provides leverage against the pushing force of delivery. As the stent 8 is delivered, distal fingers 116 of inner sheath 111 expand slightly to permit passage of a single stent out of the inner sheath (FIG. 12B). As shown in FIG. 12C, immediately after the stent 8 is delivered into the myocardium 4, the resilient fingers 116 spring back inwardly to surround and restrain the next stent from leaving the catheter 106. After delivery, the inner sheath 111 and push wire 114 are withdrawn into the catheter and the catheter is moved to a new position for the next stent delivery.

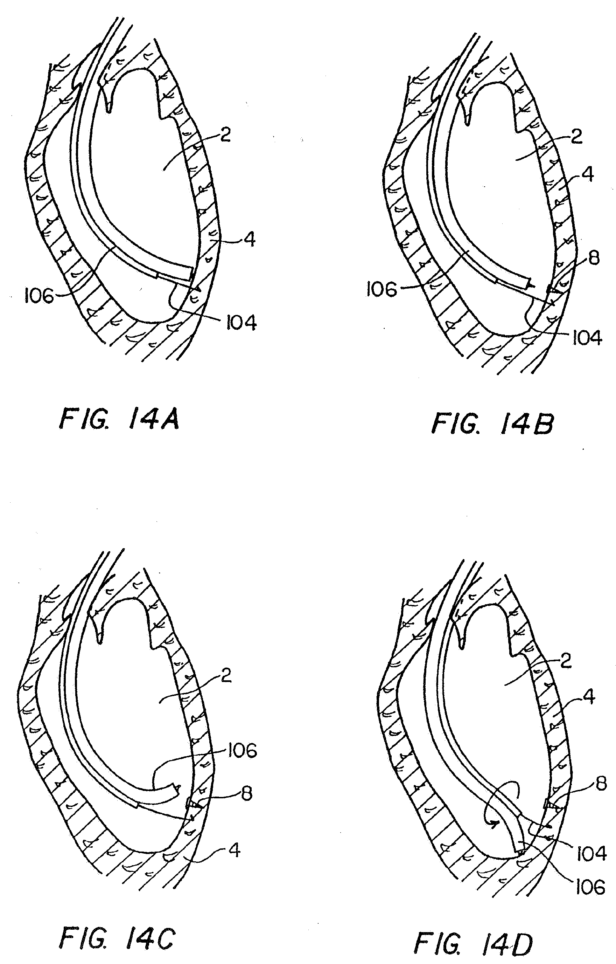

As shown in FIGS. 14A - 14D stents may be delivered sequentially to multiple sites in the myocardium through a single placement of the delivery catheter 106 within the ventricle 2. Initially, the delivery catheter 106 is advanced over the barbed tip guidewire 104 to a site on the surface of the myocardium 4. The distal tip of the delivery catheter is brought into close proximity to the myocardial tissue and a first stent is delivered in accordance with the steps described above.

As shown in FIG. 14B, after delivery of the first stent 8 into the myocardium 4, the delivery catheter 106 is withdrawn slightly in the proximal direction and is rotated about the eccentrically positioned guidewire 104 as depicted in FIGS. 14C and 14D. The distal tip of the delivery catheter 106 may also be deflected by the pull wire mechanism to provide additional range of movement. After repositioning to a new delivery site, the catheter 106 is advanced to the myocardium 4 and the next stent is delivered, again following the steps detailed above. This procedure is repeated for the delivery of the remaining stents throughout the circumferential area defined by the catheter's rotation around the eccentric guidewire. As many as ten stents or more may be delivered within such an area with a single catheterization. After delivery of the stents, the guidewire is withdrawn proximally from the myocardial tissue and the catheter and guidewire are removed from the patient.

From the foregoing, it will be appreciated that the invention provides a stent and associated delivery system for aiding revascularization of myocardial tissue of the heart. The stents are simple and readily insertable into the myocardium with a minimum of steps. The delivery system is simple to operate to implant multiple stents quickly.

It should be understood, however, that the foregoing description of the invention is intended merely to be illustrative thereof and that other modifications, embodiments, and equivalents may be apparent to those skilled in the art without departing from its spirit.

## Claims

1. A system for revascularizing myocardial tissue comprising:
a catheter;
at least one stent;
a stent delivery device configured to move the stent through the catheter and simultaneously pierce and insert the stent into myocardial tissue to be revascularized.

2. A self-piercing stent.

3. A stent as defined in claim 2 wherein the stent comprises a body having a proximal end and a distal end configured to pierce tissue.

4. A stent as defined in claim 3 wherein the body has a tubular shape.

5. A stent as defined in claim 3 wherein the body comprises a helically wrapped ribbon.

6. A stent as defined in claim 3 wherein the body is configured to be inserted into tissue as the tissue is pierced by the distal end.

7. A stent as defined in claim 3 wherein the body further comprises tissue engaging projections that engage tissue to resist migration of the stent after implantation.

8. A stent as defined in claim 6 further comprising a flange at the proximal end.

9. A stent as defined in claim 3 wherein the body defines a hollow interior.

10. A stent as defined in claim 9 wherein the body has a plurality of perfusion openings.

11. The stent as defined in claim 10 wherein a perfusion opening is located at the distal end of the stent.

12. The stent as defined in claim 2 formed of a molded polymer.

13. A revascularization device comprising:
a self-piercing stent having a body, a distal end configured to penetrate into tissue and a proximal end defining an opening into the body of the stent such that, when placed in fluid communication with a blood pathway, blood can flow into the body of the stent.

14. A stent as defined in claim 13 wherein the body comprises a molded plastic.

15. A stent as defined in claim 13 wherein the body has a tubular shape.

16. A stent as defined in claim 13 wherein the body comprises a helically wrapped ribbon.

17. A stent as defined in claim 13 wherein the body is configured to be inserted into tissue as it is pierced by the distal end.

18. A stent as defined in claim 13 wherein the body further comprises tissue engaging projections to resist migration of the stent after implantation.

19. A method of revascularizing myocardial tissue comprising:
simultaneously piercing and inserting a stent into myocardial tissue to be revascularized.

20. A method as defined in claim 19 wherein the stent is self-piercing.

21. A method as defined in claim 19 wherein the stent is inserted into the myocardium on a piercing delivery device.

22. A method as defined in claim 19 wherein the stent has a body defining a hollow interior, a distal end and a proximal end defining an opening and the opening is in communication with the left ventricle after the stent is inserted to permit blood from the ventricle to enter the interior of the stent and channel created.

23. A method as defined in claim 19 wherein the stent is inserted into only a portion of the thickness of the myocardium.

24. A method as defined in claim 19 further comprising:
introducing a delivery catheter having a distal end into the left ventricle of the heart;
positioning the distal end of the catheter adjacent myocardial tissue to be revascularized and
ejecting a stent from the distal end of the catheter to insert it into the myocardium.

25. A method as described in claim 19 wherein multiple stents are carried by the delivery catheter and are ejected sequentially, to multiple sites, in the myocardium.

26. A method of revascularizing myocardial tissue as defined in claim 25 wherein multiple stents are carried within the delivery catheter nested one partially within the other along a common longitudinal axis to define a series of nested stents having a most proximal stent and a most distal stent, such that the stents can be advanced in a distal direction by applying a delivery force on the most proximal stent to eject the most distal stent.

27. A method of revascularizing myocardial tissue as defined in claim 20 wherein the stent has a sharp distal end capable of piercing the myocardium so that the stent penetrates the tissue as a delivery force is applied.

28. A method of revascularizing myocardial tissue as defined in claim 21 wherein the piercing delivery device is joined to a distal end of a wire that is longitudinally slidable through a lumen of a catheter that has been navigated into the left ventricle of the heart adjacent myocardial tissue to be revascularized.

29. The method of revascularizing myocardial tissue as defined in claim 22 wherein the stent is defined by a body having a plurality of perfusion openings to permit blood to flow from the left ventricle through the stent and radially outward into the myocardium.

30. A nestable stent comprising:
a body defining an interior;
a proximal end defining an opening to the interior and
a distal end sized smaller than the proximal end so that when aligned with the proximal end of another nestable stent, the distal end can fit into the proximal end and at least partially into the interior of the other nestable stent.

31. A nestable stent as defined in claim 30 wherein the body comprises a molded plastic.

32. A nestable stent as defined in claim 30 wherein the body has a tubular shape.

33. A nestable stent as defined in claim 30 wherein the body comprises a helically wrapped ribbon.

34. A nestable stent as defined in claim 30 wherein the body is configured to be inserted into tissue as it is pierced by the distal end.

35. A nestable stent as defined in claim 30 wherein the body further comprises tissue engaging projections to resist migration of the stent after implantation into tissue.

36. A nestable stent as defined in claim 30 wherein the body is configured to permit blood to flow into the proximal end of the stent into the interior of the stent and then radially outward from the stent.

37. A stent delivery system comprising:
a delivery catheter having a proximal end, a distal end and at least one lumen defined between the ends;
at least one nestable stent having a body defining an interior, a proximal end defining an opening into the interior, and a distal end sized to fit within the proximal end and partially into the interior of another nestable stent, the nestable stent being sized to fit within the lumen of the catheter;
a restraint mechanism adjacent the distal end of the catheter to prevent release of a nestable stent from the catheter in the absence of an intentionally applied delivery force.

38. A stent delivery system as defined in claim 37 wherein a plurality of nestable stents are arranged within the lumen of the catheter adjacent its distal end, substantially aligned along a common longitudinal axis to define a series having a most proximal and a most distal stent such that the distal end of each stent resides within the opening at the proximal end of the next distally adjacent stent.

39. A stent delivery system as defined in claim 37 wherein the catheter is percutaneously insertable into the left ventricle of the heart through a patient's vessels.

40. A method of delivering multiple stents comprising:
providing a plurality of nestable stents each having a body defining an interior, a proximal end defining an opening into the interior, and a distal end configured to fit inside the opening at the proximal end of another nestable stent;
providing a catheter having proximal and distal ends;
disposing multiple nested stents at the distal end of the catheter such that the distal end of a stent is nested within the opening at the proximal end of an adjacent stent;
navigating the catheter to the intended stent delivery site;
ejecting each stent sequentially to its intended delivery site.

41. A method of delivering multiple stents as defined in claim 40 wherein the catheter is navigated percutaneously into the left ventricle of the heart and the stents are ejected into the myocardium.

42. A method as defined in claim 40 wherein the catheter has at least one lumen and the stents are nested along a common longitudinal axis and a catch regulates ejection of the stents so that they can be delivered sequentially.

43. A TMR stent comprising:
a body defined by a helically wrapped ribbon having a proximal end and a distal end wherein the distal end has a smaller diameter than the proximal end.

44. A TMR stent as defined in claim 43 wherein the body of the stent is flexible.

45. A TMR stent as defined in claim 43 wherein the ribbon is canted so that the ribbon presents an outwardly facing edge that engages tissue into which the stent is inserted to resist migration of the stent.

46. A TMR stent as defined in claim 45 wherein the ribbon has a rectangular cross-sectional shape.

47. A TMR stent as defined in claim 45 wherein the ribbon is wrapped to have a smaller diameter at the proximal end.

48. A TMR stent as defined in claim 43 wherein the ribbon comprises stainless steel.

49. A method of implanting a TMR stent comprising:
providing a delivery catheter having proximal and distal ends and at least one lumen disposed between the ends;
a delivery device having proximal and distal ends, longitudinally movable through the lumen of the catheter and having a piercing tip at its distal end;
providing at least one TMR stent having a body formed from a helically wrapped ribbon having proximal and distal ends;
disposing a TMR stent on the distal end of the delivery device;
navigating the catheter through vessels of a patient so that its distal end is adjacent a delivery site;
advancing the delivery device with the TMR stent distally through the catheter and out the distal end to simultaneously pierce tissue at the delivery site and implant the TMR stent in the tissue.

50. A method of delivering a TMR stent as defined in claim 49 wherein the catheter is navigated to the left ventricle of the heart and the TMR stent is implanted into the myocardium.

51. A method of delivering a TMR stent as defined in claim 49 wherein multiple TMR stents are delivered to the delivery site with one catheterization.

52. A method of delivering a TMR stent as defined in claim 49 wherein the TMR stent is flexible and the delivery device provides support for the stent during delivery into the tissue at the delivery site.

53. A method of delivering a TMR stent as defined in claim 50 wherein the distal end and body of the TMR stent are implanted into the myocardium and the proximal end of the stent is open to the left ventricle.

54. A method of navigating a catheter to multiple locations within a body comprising:
providing a steerable catheter having a distal end and at least one lumen having a central longitudinal axis;
providing a retractable piercing device at the distal end of the catheter offset from the central longitudinal axis;
inserting the distal end of the catheter into a vessel;
navigating the distal end of the catheter to an intended location;
extending the piercing device into tissue;
rotating the catheter about the piercing device and steering the distal end of the catheter to reach multiple locations in the patient.

55. A method of navigating a catheter as defined in claim 54 wherein the multiple locations reached by the catheter include a locus of points within a area defined around the location of the piercing device.

56. A system for reaching multiple points within a body comprising:
a catheter having proximal and distal ends and a first lumen extending from the proximal to the distal end, having a central longitudinal axis, and a second lumen offset from the first lumen and terminating prior to the distal end of the catheter and a pull wire joined to the distal end of the catheter and extending to the proximal end arranged to cause bending movement of the distal end of the catheter; and
a piercing guidewire slidable in the second lumen of the catheter.
